# EUROPEAN PATENT APPLICATION

(11) **EP 1 712 206 A2**
(43) Date of publication of application: **18.10.2006**
(21) Application number: 06007749.2
(22) Date of filing: 12.04.2006
(51) Int. Cl.: A61F 2/34

(54) **Acetabular implant with a tapered bearing-locking flange**

(30) Priority: 12.04.2005 US 104351
(71) Applicant: Zimmer Technology, Inc., Chicago, Illinois 60606 (US)
(72) Inventor: Newsome, Archie W., Mentone, IN 46539 (US); Schlemmer, Randy L., Bremen, IN 46506 (US)
(74) Representative: Manitz, Finsterwald & Partner GbR

(57) **Abstract**

An apparatus includes an acetabular shell 300 defining a bearing retention cavity and further defining a flange. The flange includes a first annular surface tapering inwardly into the cavity and a second annular surface extending generally radially inwardly into the cavity.

## Description

### FIELD OF THE INVENTION

The present invention relates generally to the field of orthopaedics, and, more particularly, to an acetabular implant with a tapered bearing-locking flange.

### BACKGROUND

A conventional hip prosthesis is primarily composed of an acetabular implant and a femoral implant. The acetabular implant typically includes a generally hemispherical dome-like or cup-like metallic shell secured within the acetabulum and a dome-like or cup-like plastic or ceramic bearing secured within the shell. Accordingly, the shell typically includes an exterior configured to be anchored into the acetabulum and further typically includes an interior configured to align and retain the bearing, while the bearing typically includes an exterior configured to cooperate with the interior of the shell to align and secure the bearing within the shell and further typically includes an interior defining an artificial hip socket (which may or may not be off-centered from the exterior of the bearing, depending on the particular design). The femoral implant typically includes an elongated metallic spike or post at one end and a metallic ball at the other. The post is typically configured to be anchored into the distal femoral medullary canal and the ball is typically configured to insert into the artificial socket. Pivotal freedom of the ball within the socket allows articulation of the prosthetic joint.

The capability of the acetabular implant to intra-operatively accept different bearings selectable from alternative socket orientations and/or materials is becoming an increasingly desirable feature for the hip prosthesis. However, a post-operative dissociation of the bearing from the shell can potentially degrade the biomechanics and/or wear characteristics of the prosthesis. Historically, balancing the needs for effective post-operative bearing retention with competing desires for design simplicity, versatility, and easy intra-operative bearing installation has been challenging.

### SUMMARY OF THE INVENTION

The present invention provides an apparatus including an acetabular shell defining a bearing retention cavity and further defining a flange. The flange includes a first annular surface tapering inwardly into the cavity and a second annular surface extending generally radially inwardly into the cavity.

The present invention provides an apparatus including an acetabular shell defining a bearing retention cavity. The shell further defines an annular female taper extending into the cavity, and further defines a flange outside of the taper. The apparatus further includes a bearing inserted into the cavity. The bearing defines an artificial hip socket and includes a substantially convex surface facing generally away from the socket. At least a portion of the substantially convex surface is configured to engage with the flange in opposition to dissociation of the bearing from the shell.

The present invention provides an apparatus including an acetabular shell, a bearing, first means for taper coupling the bearing to the shell, and second means, disintegrated from the first means, for opposing dissociation of the bearing from the shell.

The above-noted features and advantages of the present invention, as well as additional features and advantages, will be readily apparent to those skilled in the art upon reference to the following detailed description and the accompanying drawings, which include a disclosure of the best mode of making and using the invention presently contemplated.

### BRIEF DESCRIPTION OF THE DRAWINGS

FIG. 1 shows an exemplary hip prosthesis including an exemplary femoral implant and further including an exemplary acetabular implant according to the present invention;

FIG. 2 shows a perspective view of the exemplary acetabular implant of FIG. 1;

FIG. 3 shows an exploded axial cross-sectional view of the exemplary acetabular implant of FIG. 1;

FIG. 4 shows an enlarged exploded axial cross-sectional view of a region of the exemplary shell and a region of the exemplary bearing of the exemplary acetabular implant of FIG. 1;

FIG. 5 shows an enlarged exploded axial cross-sectional view of a region of the exemplary shell and a region of an exemplary alternative bearing;

FIG. 6 shows an enlarged assembled axial cross-sectional view of a region of the exemplary shell and a region of the exemplary bearing with the exemplary notch of FIG. 4;

FIG. 7 shows an enlarged assembled axial cross-sectional view of a region of the exemplary shell and a region of the exemplary alternative bearing with the exemplary alternative notch of FIG. 5;

FIG. 8 shows an enlarged assembled axial cross-sectional view of a region of an alternative shell and bearing with an alternative notch and flange arrangement; and

FIG. 9 shows an enlarged assembled axial cross-sectional view of a region of the shell of FIG. 8 and an alternative bearing arrangement.

### DETAILED DESCRIPTION OF EXEMPLARY EMBODIMENT(S)

Like reference numerals refer to like parts throughout the following description and the accompanying drawings. As used herein, the terms "medial," "medially," and the like mean pertaining to the middle, in or toward the middle, and/or nearer to the middle of the body when standing upright. Conversely, the terms "lateral," "laterally," and the like are used herein as opposed to medial. For example, the medial side of the knee is the side closest to the other knee and the closest sides of the knees are medially facing, whereas the lateral side of the knee is the outside of the knee and is laterally facing. Further, as used herein the term "superior" means closer to the top of the head and/or farther from the bottom of the feet when standing upright. Conversely, the term "inferior" is used herein as opposed to superior. For example, the heart is superior to the stomach and the superior surface of the tongue rests against the palate, whereas the stomach is inferior to the heart and the palate faces inferiorly toward the tongue. Also, as used herein the terms "anterior," "anteriorly," and the like mean nearer the front or facing away from the front of the body when standing upright, as opposed to "posterior," "posteriorly," and the like, which mean nearer the back or facing away from the back of the body. Additionally, as used herein the term "generally hemispherical" is intended its broadest sense to encompass all concave and convex geometries suitable for applicable components of prosthetic ball-and-socket type joints such as acetabular and glenoid shells, integuments, bearings, and the like, and, accordingly, includes hemispherical geometries, includes partially spherical geometries that are more than hemispherical, includes partially spherical geometries that are less than hemispherical, and includes all suitable curved polygonal and geodesic geometries as well. Further, as used herein the term "taper" and inflections thereof are intended in their broadest sense to mean to become gradually slenderer or less in diameter, while the terminology "taper couple" and inflections thereof mean to fasten together via a taper joint. In general, a taper joint or taper coupling is formed by pressing together ("press-fitting") a male part ("male taper") and a female part ("female taper") having impinging angled or flared surfaces. Various taper couplings are generally known in the art. For example, the disclosure of United States Patent No. 6,610,097 to Serbousek et al, which is expressly incorporated herein by reference, discusses manners of making and using various taper couplings that may be suitable for incorporation into applicable embodiments of the present invention.

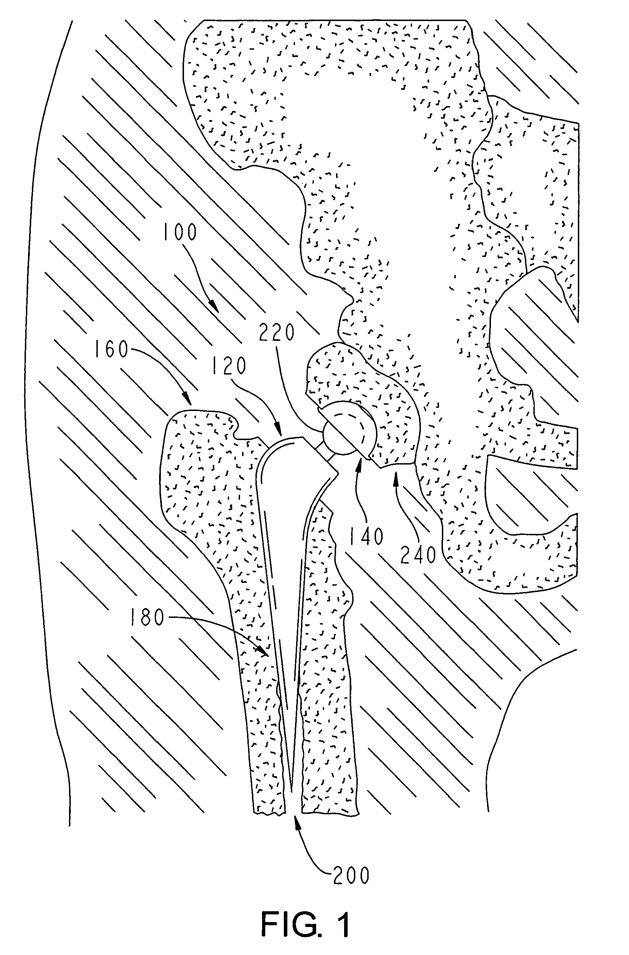

FIG. 1 shows an exemplary hip prosthesis 100 including an exemplary femoral implant 120 and further including an exemplary acetabular implant 140 according to the present invention. Among other things, implant 120 is configured as known to replace natural hip components (not shown) of a distal femur 160. In the exemplary embodiment, implant 120 is metallic and preferably made from titanium. In alternative embodiments, implant 120 may be made from a cobalt chrome alloy or any other suitable biocompatible material(s). Implant 120 includes a post 180. Among other things, post 180 is configured as known to anchor into a medullary canal 200 of distal femur 160. Implant 120 also includes a substantially spherical ball 220.

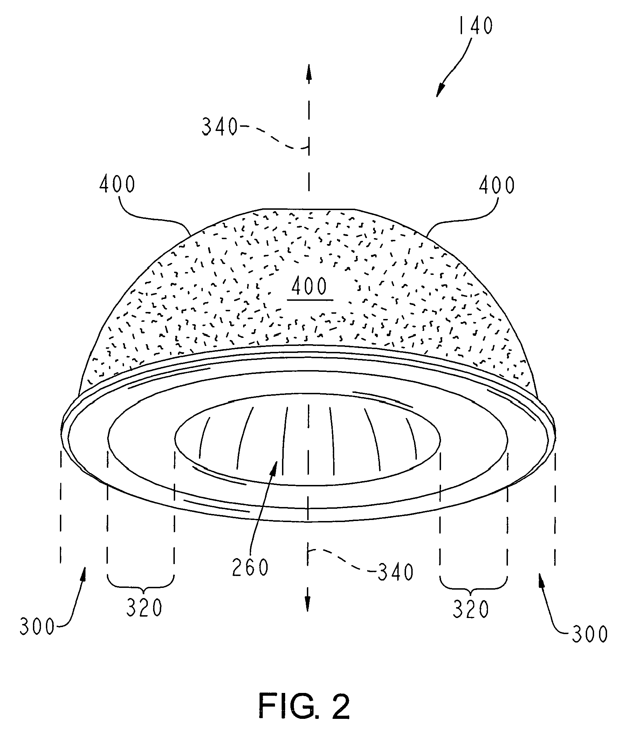

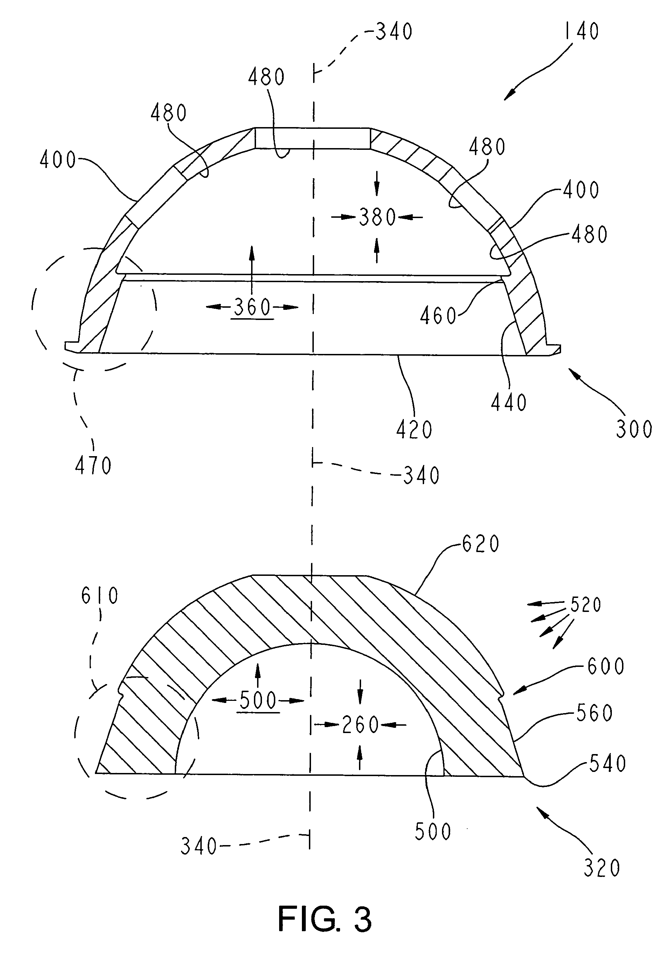

Among other things, implant 140 is configured to replace natural hip components (not shown) of an acetabulum 240. Accordingly, implant 140 defines a generally hemispherical artificial hip socket 260 (see FIG. 2 and FIG. 3) that receives ball 220 as known such that ball 220 has suitable pivotal freedom within socket 260. Implant 140 is discussed further below.

FIG. 2 shows a perspective view of exemplary implant 140. Implant 140 includes a dome-like or cup-like acetabular shell 300, and a dome-like or cup-like bearing 320. Among other things, shell 300 is configured to be anchored into acetabulum 240 (see FIG. 1) in a known manner, and is configured to couple to bearing 320 in accordance with the exemplary embodiment of the present invention. In the exemplary embodiment, shell 300 is metallic and preferably made from titanium. In alternative embodiments, shell 300 may be made from a cobalt chrome alloy or any other suitable biocompatible material(s). Further, shell 300 is symmetrical about an axis 340 and includes a substantially concave inner surface 360 (see FIG. 3) defining a substantially concave bearing retention cavity 380 (see FIG. 3) that is symmetrical about axis 340. Further, shell 300 includes a generally hemispherical outer surface 400 facing generally outwardly away from socket 380. In the exemplary embodiment, surface 400 is suitably textured as known to facilitate fixation in acetabulum 240. Additionally, it is noted that surface 400 may be suitably covered with a porous material (not shown) as known to enhance acetabular fixation of shell 300 through bone in growth. Also, it is noted that in alternative embodiments one or more holes or apertures (not shown) may pass through shell 300 to allow additional acetabular fixation of shell 300 as known with bone screws, nails, or the like.

Among other things, bearing 320 is configured as known to receive ball 220 (see FIG. 1) in socket 260 and is configured to couple to shell 300 according to the exemplary embodiment of the present invention. In one embodiment, bearing 320 is made from a plastic, preferably ultra high molecular weight polyethylene ("UHMWPE"). In alternative embodiments, bearing 320 may be made from a ceramic, metal, and/or any other suitable biocompatible material(s). Bearing 320 is discussed further below.

FIG. 3 shows an exploded axial cross-sectional view of implant 140. As at least partially discernable in FIG. 3, surface 360 of shell 300 includes an annular rim 420. Cavity 380 opens at rim 420. Further, surface 360 defines an annular female taper 440 extending into cavity 380 from rim 420. Surface 360 also defines an annular tapered flange 460 extending from taper 440 in an annular area or region 470. Among other things, flange 460 is configured to engage with bearing 320 in opposition to dissociation of bearing 320 from shell 300 according to the exemplary embodiment. It is noted that flange 460 is positioned axially inward in cavity 380 relative to taper 440. Flange 460 is discussed further below. Additionally, surface 360 includes a generally hemispherical concave portion 480 extending from flange 460 and inwardly bounding cavity 360.

Bearing 320 includes a generally hemispherical and substantially concave inner surface 500 that is suitably machined as known to define socket 260. Bearing 320 also includes a substantially convex outer surface 520 that faces generally outwardly away from socket 260. Surface 520 includes an annular rim 540 and defines an annular male taper 560 extending from rim 540. Taper 560 is configured as known to suitably press-fit into and taper couple to taper 440 (of shell 300). Surface 520 also defines an annular tapered notch 600 extending from taper 560 in an annular area or region 610. Among other things, notch 600 is configured to engage with flange 460 in opposition to dissociation of bearing 320 from shell 300 according to the exemplary embodiment. Notch 600 is discussed further below. Additionally, surface 520 includes a generally hemispherical convex portion 620 extending from notch 600. Among other things, portion 620 is configured to be suitably retained in cavity 380 (of shell 300) axially inwardly of flange 460 when bearing 320 is press-fitted into shell 300.

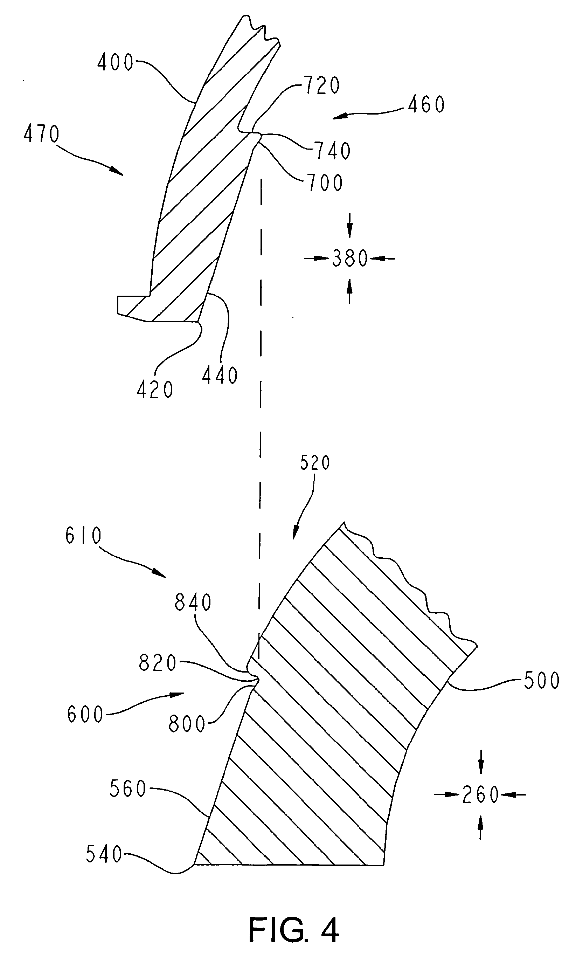

FIG. 4 shows an enlarged exploded axial cross-sectional view of region 470 (of shell 300) and region 610 (of bearing 320). As at least partially discernable in FIG. 4, flange 460 includes an annular surface 700 tapering inwardly into cavity 380 (see also FIG. 3), further includes an annular surface 720 extending generally radially inwardly into cavity 380, and further includes an annular surface 740 convexly curling or rounding between surface 700 and surface 720. Meanwhile, surface 520 defines notch 600 with a portion 800 tapering inwardly towards socket 260 (see also Fig. 3), further defines notch 600 with a portion 820 curling concavely from portion 800 (see also Fig. 3), and further defines notch 600 with a portion 840 curling convexly from portion 800 (see also Fig. 3). Rim 420, taper 440, rim 540, and taper 560, among other things, are also all at least partially discernable in FIG. 4. It is noted that flange 460 (and/or any of its alternative embodiments) preferably is outside or disintegrated from (as opposed to interposed within) taper 440 (and/or any of its alternative embodiments) and notch 600 (and/or any of its alternative embodiments) preferably is outside or disintegrated from (as opposed to interposed within) taper 560 (and/or any of its alternative embodiments) such that taper 440, flange 460, taper 560, and notch 600 (and/or any of their alternative embodiments) preferably are mutually positioned and mutually configured such that extension of flange 460 into notch 600 (and/or any of their alternative embodiments) engages flange 460 with notch 600 (and/or any of their alternative embodiments) in opposition to dissociation of bearing 320 from shell 300 without significantly interrupting, forcing apart, or otherwise compromising the taper coupling between taper 440 and taper 560 (and/or any of their alternative embodiments).

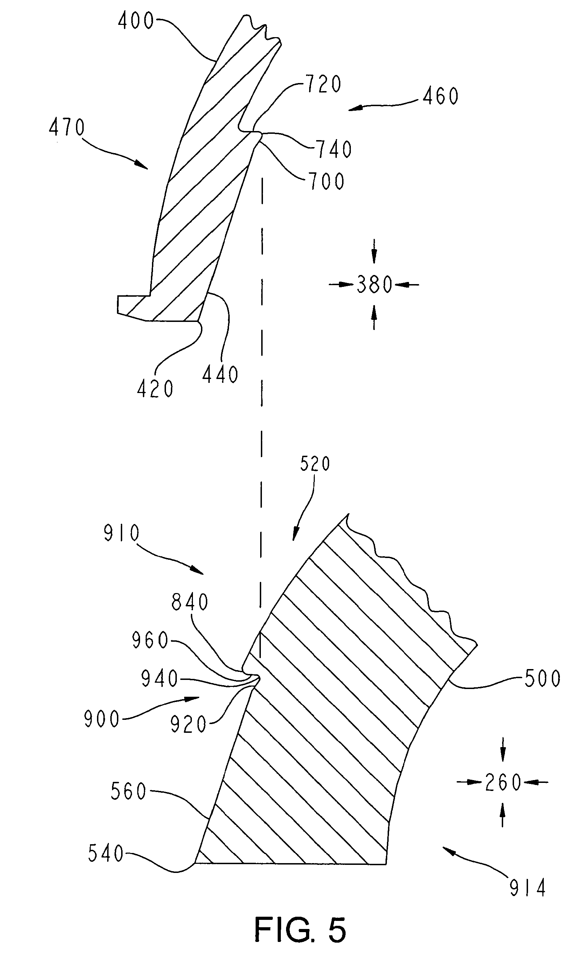

FIG. 5 shows an enlarged exploded axial cross-sectional view of region 470 (of shell 300) and a region 910 of an alternative bearing 914. Bearing 914 is configured in a like manner to bearing 320 with the exception that notch 600 is replaced with an alternative deeper notch 900 defined by portion 840, by an alternative surface portion 920 tapering inwardly towards socket 260, by an alternative surface portion 940 curling concavely from portion 920, and by a radially inwardly extending portion 960 interposed between portion 940 and portion 840.

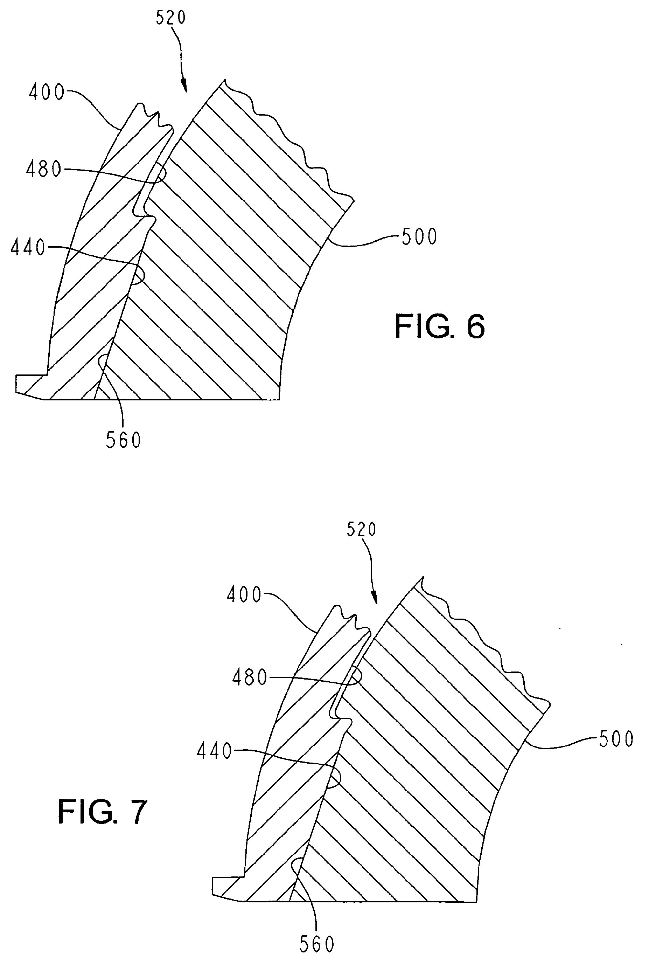

FIG. 6 shows an enlarged assembled axial cross-sectional view of region 470 (of shell 300) and region 610 (of bearing 320) with notch 600. Surface 400, taper 440, portion 480, surface 500, surface 520, and taper 560, among other things, are all at least partially discernable in FIG. 6. To assemble prosthesis 100, distal femur 160 and acetabulum 240 are suitably resected, post 180 is suitably anchored into medullary canal 200, and shell 300 is suitably anchored into acetabulum 240. Bearing 320 is suitably rotationally aligned relative to shell 300 and then press-fitted into socket 380 (of shell 300) such that taper 440 taper couples to taper 560 and flange 460 extends into and engages with notch 600 in opposition to dissociation of bearing 320 from shell 300. Lastly, ball 220 is inserted into socket 260. In operation of prosthesis 100, bearing 320 stays coupled to shell 300 within socket 380. Further, pivotal freedom of ball 220 within socket 260 allows articulation of implant 120 relative to implant 140.

It should be appreciated that as bearing 320 is press-fitted into shell 300, portion 840 of surface 520 (of bearing 320) progressively slides axially and radially inwardly along surface 700 (of flange 460) until bearing 320 and shell 300 are pressed together tightly enough for surface 720 (of flange 460) to clear portion 840. The tapered design of flange 460 thereby facilitates the engagement of flange 460 and notch 600 and generally reduces the insertion forces required to lock bearing 320 into shell 300. Meanwhile, the radial seating of surface 720 against portion 820 of surface 520 (and slightly axially outwardly of portion 840) effectively locks bearing 320 into shell 300 upon engagement of flange 460 and notch 600. Additionally, flange 460 engages notch 600 without significantly interrupting, forcing apart, or otherwise compromising the taper coupling between taper 440 and taper 560. Thus, among other things, the present invention offers easy intra-operative bearing installation, design simplicity, and effective post-operative bearing retention. Moreover, it is noted that the positioning of flange 460 and notch 600 axially inwardly of taper 440 and taper 560, respectively, allows the taper coupling between taper 440 and taper 560 to block wear debris (which might be generated by the engagement of flange 460 and notch 600) from escaping around rim 420 and rim 540.

FIG. 7 shows an enlarged assembled axial cross-sectional view of region 470 (of shell 300) and region 910 (of alternative bearing 914) with notch 900. Surface 400, taper 440, portion 480, surface 500, surface 520, and taper 560, among other things, are all at least partially discernable in FIG. 7. It should be appreciated that embodiments employing notch 900 are assembled and operated in a like manner to embodiments employing notch 600 (discussed above).

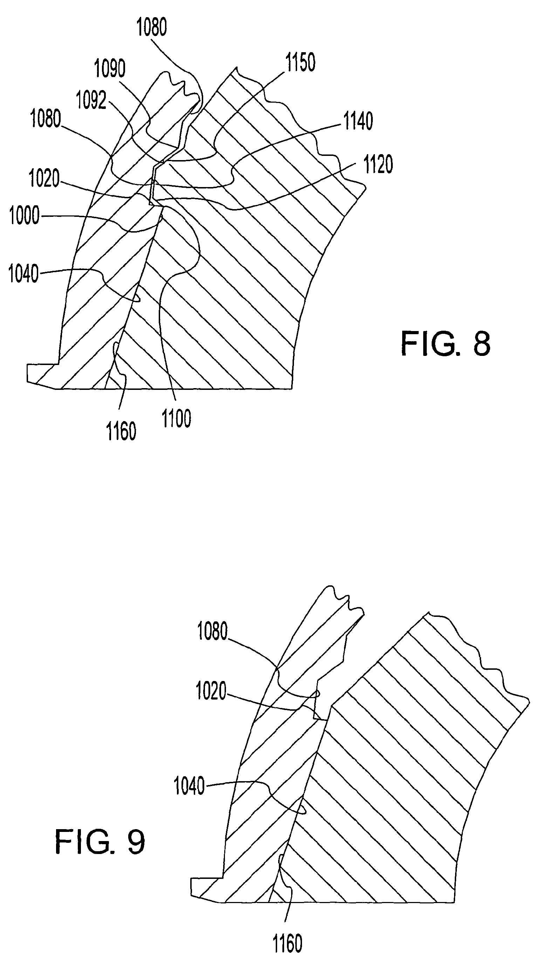

FIG. 8 shows an enlarged assembled axial cross-sectional view of an alternative engagement arrangement between shell 300 and bearing 320. The shell 300 includes a female taper 1040 terminating at an annular surface 1000 continuous and flush with the taper 1040 as opposed to the curling surface 740 of FIG. 4. Annular surface 1020 extends generally radially outwardly from annular surface 1000. Concave portion 1080 extends from annular surface 1020. An annular ridge 1090 projects inwardly from concave portion 1080 and provides an abutment surface 1092 opposing the bearing 320. Bearing 320 includes a male taper 1160 engageable with the female taper 1040. The curved notch 800 of FIG. 4 is replaced with a sharp corner 1100 where the male taper 1160 meets an angled surface 1120. The angled surface 1120 bounds the lower side of a portion 1140 overhanging the corner 1100. The portion 1140 is bounded on its top by top surface 1150. The portion 1140 snaps into concave portion 1080 above annular surface1020 upon insertion of the bearing 320 into the shell 300. The boxy geometry of portion 1140 results in an increased cross sectional area and an increased shear area than the configuration of FIG. 4. The overall effect is to increase the strength of the snap-fit engagement. The top surface 1150 is opposed by surface 1092 of the annular ridge 1090 to reduce pistoning of the bearing 320 within the shell 300. By locating the annular ridge 1090 in close proximity to the snap mechanism, the portion of the assembly for which close machining tolerance is required is confined in one area and more easily manufactured.

FIG. 9 shows an alternative bearing 320 omitting the snap mechanism and relying solely on the engagement of tapers 1040 and 1160 for retaining the bearing 320 in the shell 300. This is particularly useful where the bearing 320 is made of a rigid material unsuitable for a snap mechanism. For example, a bearing 320 made of ceramic, metal, and/or other rigid materials is unable to flex sufficiently to facilitate a snap arrangement but is well suited to a rigid locking taper arrangement. The surface 1020, concave portion 1020, and other elements of the shell-side snap mechanism are located axially inwardly of the taper surfaces 1040, 1160 so that they do not interrupt the taper surfaces or otherwise limit their taper locking engagement. Thus, the shell 300 of the present invention can accommodate both resilient bearings 320 having a snap-lock and/or a taper lock engagement and rigid bearings 320 having taper lock engagement.

The foregoing description of the invention is illustrative only, and is not intended to limit the scope of the invention to the precise terms set forth. Further, although the invention has been described in detail with reference to certain illustrative embodiments, variations and modifications exist within the scope and spirit of the invention as described and defined in the following claims.

## Claims

1. An apparatus, comprising:
an acetabular shell (300);
a first bearing (320);
first means for taper coupling the first bearing (320) to the shell (300); and
second means, disintegrated from the first means, for opposing dissociation of the first bearing (320) from the shell (300).

2. The apparatus of claim 1, **characterized in that** the shell (300) defines a cavity (380) and the second means is positioned at least axially inward in the cavity (380) relative to the first means.

3. The apparatus of claim 1 or 2, **characterized in that** the acetabular shell (300) defines a bearing retention cavity (380) and the second means comprises a flange (460) including a first annular surface (700) tapering inwardly into the cavity (380) and a second annular surface (720) extending generally radially inwardly into the cavity (380).

4. The apparatus according to any of the preceding claims, **characterized in that** the shell (300) further defines an annular female taper (440) extending into the cavity (380) and the flange (460) is positioned at least axially inward in the cavity (3 80) relative to the female taper (400).

5. The apparatus according to any of the preceding claims, **characterized in that** the flange (460) further includes a third annular surface (740) curling between the first annular surface (700) and the second annular surface (720).

6. The apparatus according to any of the preceding claims, **characterized in that** the first bearing (320) defines an artificial hip socket (260) and includes a substantially convex surface (520) facing generally away from the socket (260), at least a portion of the substantially convex surface (520) being configured to engage with the flange (460) in opposition to dissociation of the first bearing (320) from the shell (300).

7. The apparatus according to any of the preceding claims, **characterized in that** the first bearing (320) further defines an annular male taper (560) and the female taper (440) is taper coupled to the male taper (560).

8. The apparatus according to any of the preceding claims, **characterized in that** the at least a portion of the substantially convex surface (520) includes a first portion (800) tapering inwardly towards the socket (260).

9. The apparatus of claim 8, **characterized in that** the at least a portion of the substantially convex surface (520) further includes a second portion (820) curling from the first portion (800).

10. The apparatus according to any of the preceding claims,
**characterized in that**
it further comprises a femoral implant (120) including a ball (220) positioned in the socket (260).

11. The apparatus according to any of the preceding claims, **characterized in that** the first bearing (320) comprises a relatively resilient material and the second means comprises a snap-fit engagement, the apparatus further comprising an alternative second bearing (320) comprising a relatively rigid material, the second bearing (320) being engageable with the shell (300) alternatively with the first bearing (320), the second bearing (320) being engageable with the first means for taper coupling the first bearing (320) to the shell (300).
